(19)

Europäisches Patentamt

European Patent Office

Office européen des brevets

(11) **EP 3 263 212 A1**

(12) **EUROPEAN PATENT APPLICATION**
published in accordance with Art. 153(4) EPC

(43) Date of publication:
**03.01.2018 Bulletin 2018/01**

(21) Application number: **16755218.1**

(22) Date of filing: **10.02.2016**

(51) Int Cl.:
*B01J 23/30* (2006.01)     *B01J 37/04* (2006.01)
*C07C 1/20* (2006.01)      *C07C 11/167* (2006.01)
*C07B 61/00* (2006.01)

(86) International application number:
**PCT/JP2016/053948**

(87) International publication number:
**WO 2016/136471 (01.09.2016 Gazette 2016/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **27.02.2015 JP 2015038403**

(71) Applicant: **Sekisui Chemical Co., Ltd.**
**Osaka-shi, Osaka 530-8565 (JP)**

(72) Inventors:
• **NISHINO Tomoaki**
  **Tsukuba-shi**
  **Ibaraki 300-4292 (JP)**
• **MIYAMA Toshihito**
  **Tsukuba-shi**
  **Ibaraki 300-4292 (JP)**

(74) Representative: **Merkle, Gebhard**
**Ter Meer Steinmeister & Partner**
**Patentanwälte mbB**
**Nymphenburger Straße 4**
**80335 München (DE)**

(54) **CATALYST FOR 1, 3-BUTADIENE SYNTHESIS, METHOD FOR PRODUCING CATALYST FOR 1, 3-BUTADIENE SYNTHESIS, APPARATUS FOR PRODUCING 1, 3-BUTADIENE, AND METHOD FOR PRODUCING 1, 3-BUTADIENE**

(57)     [1] A catalyst for synthesizing 1,3-butadiene by contact with ethanol, which comprises tungsten oxide and magnesium oxide. [2] The catalyst, wherein a mass ratio of the magnesium oxide to the tungsten oxide (magnesium oxide / tungsten oxide) is 0.1 to 200. [3] The catalyst, wherein the mass ratio is at least 5. [4] The catalyst, wherein amounts of the tungsten oxide and the magnesium oxide relative to 100% by mass of the catalyst are as follows: the amount of the tungsten oxide: 0.1 to 90 % by mass; and the amount of the magnesium oxide: 10 to 90 % by mass.

FIG. 1

**Description**

TECHNICAL FIELD

The present invention relates to a catalyst for synthesizing 1,3-butadiene, a method for producing the catalyst for synthesizing 1,3-butadiene, an apparatus for producing 1,3-butadiene, and a method for producing 1,3-butadiene.
Priority is claimed on Japanese Patent Application No. 2015-038403, filed February 27, 2015, the contents of which are incorporated herein by reference.

DESCRIPTION OF RELATED ART

1,3-Butadiene is one of the C4 compounds important in chemical industry, which are used as raw materials for synthetic rubbers such as styrene-butadiene rubber (SBR) (see, for example, Patent Document 1). Conventionally, butadiene is obtained by purifying a C4 fraction by-produced during the synthesis of ethylene from petroleum. However, recently, a method is proposed in which butadiene is produced from a biomass-derived bioethanol in the presence of a catalyst (see, for example, Patent Document 2).

DOCUMENTS OF RELATED ART

[Patent Document]

[Patent Document 1] Japanese Unexamined Patent Application Publication No. 2013-53211
[Patent Document 2] International Patent Application Publication No.2014/129248

DISCLOSURE OF INVENTION

PROBLEMS TO BE SOLVED BY THE INVENTION

However, a catalyst which enables 1,3-butadiene to be synthesized more efficiently has been demanded.
The present invention has been made in view of the above situation, and the object of the present invention is to provide a catalyst which enables 1,3-butadiene to be synthesized more efficiently from a gaseous raw material containing ethanol, a method for producing said catalyst, an apparatus for producing said catalyst, and a method for producing 1,3-butadiene by the use of said catalyst.

MEANS TO SOLVE THE PROBLEMS

1. A catalyst for synthesizing 1,3-butadiene by contact with ethanol, which includes tungsten oxide and magnesium oxide.
2. The catalyst according to item 1 above, wherein a mass ratio of the magnesium oxide to the tungsten oxide (magnesium oxide / tungsten oxide) is 0.1 to 200.
3. The catalyst according to item 1 or 2 above, wherein the mass ratio is at least 5.
4. The catalyst according to any one of items 1 to 3 above, wherein amounts of the tungsten oxide and the magnesium oxide relative to 100% by mass of the catalyst are as follows:

    the amount of the tungsten oxide: 0.1 to 90 % by mass; and
    the amount of the magnesium oxide: 10 to 90 % by mass.

5. The catalyst according to any one of items 1 to 3 above, which further includes at least one inorganic oxide other than tungsten oxide and magnesium oxide.
6. The catalyst according to item 5 above, wherein an amount of the inorganic oxide other than tungsten oxide and magnesium oxide is 0.1 to 89.9 % by mass relative to 100 % by mass of the catalyst.
7. The catalyst according to item 6 above, wherein the amounts of the tungsten oxide, the magnesium oxide, and the inorganic oxide other than tungsten oxide and magnesium oxide relative to 100% by mass of the catalyst are as follows:

the amount of the tungsten oxide: 0.1 to 89.9 % by mass;
the amount of the magnesium oxide: 10 to 90 % by mass; and
the amount of the inorganic oxide other than tungsten oxide and magnesium oxide: 0.1 to 89.9 % by mass.

8. The catalyst according to item 6 or 7 above, wherein the inorganic oxide other than tungsten oxide and magnesium oxide is silicon dioxide.

9. A method for producing the catalyst according to any one of items 1 to 8 above, including: a step of mixing said oxides for forming the catalyst or precursors of said oxides in a dispersion medium or a solvent to thereby obtain a dispersion or a solution; a step of heating and drying the obtained dispersion or solution; and a step of calcining a resultant dried product.

10. An apparatus for producing 1,3-butadiene, including: a reaction tube filled with the catalyst according to any one of items 1 to 8 above; a supply means for supplying ethanol into the reaction tube; and a withdrawal means for withdrawing a reaction product from the reaction tube.

11. A method for producing 1,3-butadiene, comprising contacting ethanol with the catalyst according to any one of items 1 to 8 above.

In the present invention, the performance of a catalyst for synthesizing 1,3-butadiene is evaluated based on criteria such as the ethanol-conversion, the selectivity for 1,3-butadiene, and the yield of 1,3-butadiene.

In the present specification, the "ethanol conversion" is a molar percentage of ethanol consumed in the synthesis of various products including 1,3-butadiene, relative to the total amount of ethanol in the gaseous raw material.

Further, the "selectivity" means a molar percentage of carbon (C) converted to a specific product, relative to the total amount of carbon (C) included in the consumed ethanol in the gaseous raw material. For example, when all of the ethanol molecules consumed in the gaseous raw material are consumed in the reaction represented by the following formula ($\alpha$), the selectivity for 1,3-butadiene is 100 mol%.

$$2C_2H_5OH \rightarrow H_2C=CH\text{-}CH=CH_2+2H_2O+H_2 \cdots \qquad (\alpha)$$

EFFECT OF THE INVENTION

By using the catalyst for synthesizing 1,3-butadiene of the present invention and the method and the apparatus for producing 1,3-butadiene using said catalyst, 1,3-butadiene can be synthesized more efficiently from the gaseous raw material containing ethanol. Further, by the method of the present invention for producing said catalyst of the present invention, a catalyst having excellent catalytic activity can be obtained.

BRIEF DESCRIPTION OF THE DRAWINGS

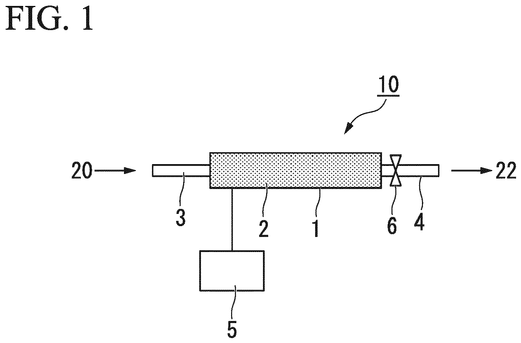

FIG. 1 is a schematic view of the apparatus for producing 1,3-butadiene according to an embodiment of the present invention.

DESCRIPTION OF THE EMBODIMENTS

<Catalyst for synthesizing 1,3-butadiene>

The catalyst for synthesizing 1,3-butadiene (hereinafter, sometimes simply referred to as "synthesis catalyst") of the present invention is a catalyst used for synthesizing 1,3-butadiene while being in contact with ethanol, which includes tungsten oxide and magnesium oxide as catalyst components.

The synthesis catalyst of the present invention may either in the form of a supported catalyst which is supported on a porous carrier to be described below, or in the form in which the oxides forming the catalyst are held, joined or aggregated together. The synthesis catalysts in unsupported form are collectively referred to as "unsupported-type composite oxide catalyst".

In the present embodiment, in order to improve the efficiency of 1,3-butadiene synthesis, tungsten oxide is used. Tungsten oxide is considered to act as a promoter in the catalytic reaction to obtain 1,3-butadiene from ethanol, which results in the improvement of the selectivity for 1,3-butadiene.

Further, in the present embodiment, from the viewpoint of improving the efficiency of 1,3-butadiene synthesis, magnesium oxide is used. Magnesium oxide is considered to be an active species in the catalytic reaction to obtain 1,3-butadiene from ethanol.

Furthermore, in the present embodiment, in order to further improve the efficiency of synthesizing 1,3-butadiene as a target product, both of tungsten oxide and magnesium oxide are used in combination.

The specific surface area of the synthesis catalyst is not particularly limited; however, in order to improve the efficiency of the target product synthesis, for example, the specific surface area is preferably 1 to 1000 $m^2/g$, more preferably 300 to 800 $m^2/g$, still more preferably 400 to 700 $m^2/g$.

The specific surface area means the BET specific surface area measured by the BET gas adsorption method using nitrogen as an adsorption gas.

The mass ratio of the magnesium oxide to the tungsten oxide (magnesium oxide / tungsten oxide) of the synthesis catalyst is not particularly limited; however, in order to improve the efficiency of the target product synthesis, for example, the mass ratio is preferably 0.1 to 200, more preferably 1 to 100, still more preferably 5 to 50.

The mass ratio of not below the above-mentioned lower limit of 0.1 can further enhance the above-mentioned effect attributable to the use of magnesium oxide. The mass ratio not exceeding the above-mentioned upper limit of 200 can further enhance the above-mentioned effect attributable to the use of tungsten oxide.

In the synthesis catalyst, it is preferable that the magnesium oxide is present in an amount 5 times or more, more preferably 10 times or more, more preferably 15 times or more the amount of tungsten oxide on a mass basis.

In addition, the amount of the magnesium oxide is preferably less than 30 times, more preferably less than 25 times, still more preferably less than 20 times the amount of tungsten oxide on a mass basis.

When the amounts of magnesium oxide and tungsten oxide are within the above ranges, the synthesis efficiency of the target product can be further improved.

The preferable ranges of the amounts of the tungsten oxide and the magnesium oxide relative to 100 % by mass of the synthesis catalyst are as follows:

> the amount of the tungsten oxide: 0.1 to 90 % by mass; and
> the amount of the magnesium oxide: 10 to 90 % by mass.

The more preferable ranges of the amounts of the tungsten oxide and the magnesium oxide relative to 100 % by mass of the synthesis catalyst are as follows:

> the amount of the tungsten oxide: 1.0 to 45 % by mass; and
> the amount of the magnesium oxide: 30 to 90 % by mass.

The still more preferable ranges of the amounts of the tungsten oxide and the magnesium oxide relative to 100 % by mass of the synthesis catalyst are as follows:

> the amount of the tungsten oxide: 2.0 to 20 % by mass; and
> the amount of the magnesium oxide: 50 to 90 % by mass.

The most preferable ranges of the amounts of the tungsten oxide and the magnesium oxide relative to 100% by mass of the synthesis catalyst are as follows:

> the amount of the tungsten oxide: 3.0 to 10 % by mass; and
> the amount of the magnesium oxide: 70 to 90 % by mass.

When the amounts of the tungsten oxide and the magnesium oxide are within the above ranges, the synthesis efficiency of the target product can be further improved.

The amounts of the tungsten oxide and the magnesium oxide present in the synthesis catalyst of the present invention reflect the compounding ratio of tungsten and magnesium which are blended during the production of the catalyst. The amount of the tungsten oxide contained in the synthesis catalyst is calculated in terms of $WO_3$, and the amount of the magnesium oxide is calculated in terms of MgO.

A method for measuring the concentrations of elements present in the synthesis catalyst of the present invention is not particularly limited, and for example, a general elemental analysis method using a known composite oxide catalyst as a sample can be used. Specifically, for example, the fluorescent X-ray analysis (XRF) is preferable. Alternatively, the measurement can be done by the inductively coupled plasma (ICP) emission spectrometry using, as a sample, a solution obtained by dissolving the synthesis catalyst into an appropriate solvent.

With respect to the molar ratio of elements other than oxygen present in the synthesis catalyst, the molar ratio of the magnesium to the tungsten (Mg / W) is preferably in the range of from 500/1 to 10/1, more preferably from 300/1 to 30/1, still more preferably from 150/1 to 60/1.

When the amounts of magnesium and tungsten are within the above ranges, the synthesis efficiency of the target product can be further improved.

It is preferred that the synthesis catalyst further includes one or more inorganic oxides other than tungsten oxide and magnesium oxide (hereinafter, sometimes referred to simply as "inorganic oxide ZO").

Here, the above-mentioned inorganic oxide ZO is preferably a substance which directly or indirectly contributes to the increase in catalytic activity (that is, a substance having catalytic activity) by serving as a binder for bonding tungsten oxide and magnesium oxide to each other.

The incorporation of the above-mentioned inorganic oxide ZO into the synthesis catalyst can further improve the efficiency of the target product synthesis.

Hereinafter, the amount of the above-mentioned inorganic oxide ZO in the synthesis catalyst embraces the total amount of two or more types of the above-mentioned inorganic oxide ZO when present in the synthesis catalyst, unless otherwise specified

In the synthesis catalyst, the amount of the above-mentioned inorganic oxide ZO is preferably 1.5 times or more, more preferably 2.0 times or more, still more preferably 2.5 times or more the amount of the tungsten oxide on a mass basis.

In addition, the amount of the inorganic oxide ZO is preferably less than 15 times, more preferably less than 10 times, still more preferably less than 5 times the amount of the tungsten oxide on a mass basis.

The presence of the above-mentioned inorganic oxide ZO in an amount within the above range can further improve the efficiency of the target product synthesis.

In the synthesis catalyst, the amount of the above-mentioned inorganic oxide ZO is preferably 0.05 times or more, more preferably 0.1 times or more, still more preferably 0.15 times or more the amount of magnesium oxide on a mass basis.

In addition, the amount of the inorganic oxide ZO is preferably less than 0.6 times, more preferably less than 0.4 times, still more preferably less than 0.2 times the amount of the magnesium oxide on a mass basis.

The presence of the above-mentioned inorganic oxide ZO in an amount within the above range can further improve the efficiency of the target product synthesis.

The amount of the inorganic oxide ZO in the synthesis catalyst relative to 100% by mass of the catalyst is preferably 0.1 to 89.9 % by mass, more preferably 1.0 to 50 % by mass, still more preferably 5.0 to 30 % by mass, most preferably 10 to 20 % by mass. Further, when the amount of the above-mentioned inorganic oxide ZO is 0.1 % or more by mass, preferable amount of the tungsten oxide is 89.9 % or less by mass.

The presence of the above-mentioned inorganic oxide ZO in an amount within the above range can further improve the efficiency of the target product synthesis.

With respect to the molar ratio of elements forming the synthesis catalyst other than oxygen, the molar ratio (Mg / Z) of the magnesium to Z (i.e., at least one inorganic element other than oxygen, which forms the above-mentioned inorganic oxide ZO) is preferably in the range of from 50/1 to 1/1, more preferably from 30/1 to 3/1, still more preferably from 15/1 to 5/1.

When the amounts of Z and tungsten are within the above ranges, the synthesis efficiency of the target product can be further improved.

With respect to the molar ratio of elements forming the synthesis catalyst other than oxygen, the molar ratio (Z / W) of Z (i.e., one or more elements other than oxygen, which form the above-mentioned inorganic oxide) to the tungsten is preferably in the range of from 50/1 to 1/1, more preferably from 30/1 to 3/1, still more preferably from 15/1 to 5/1. When the amounts of Z and tungsten are within the above ranges, the synthesis efficiency of the target product can be further improved.

The preferable ranges of the amounts of the tungsten oxide, the magnesium oxide and the above-mentioned inorganic oxide ZO relative to 100 % by mass of the synthesis catalyst are as follows:

the amount of the tungsten oxide: 0.1 to 89.9 % by mass;
the amount of the magnesium oxide: 10 to 90 % by mass; and
the amount of the inorganic oxide ZO: 0.1 to 89.9 % by mass.

The more preferable ranges of the amounts of the tungsten oxide, the magnesium oxide and the above-mentioned inorganic oxide ZO relative to 100% by mass of the synthesis catalyst are as follows:

the amount of the tungsten oxide: 1.0 to 45 % by mass;
the amount of the magnesium oxide: 30 to 90 % by mass; and
the amount of the inorganic oxide ZO: 1.0 to 65 % by mass.

The still more preferable ranges of the amounts of the tungsten oxide, the magnesium oxide and the above-mentioned inorganic oxide ZO relative to 100% by mass of the synthesis catalyst are as follows:

the amount of the tungsten oxide: 2.0 to 20 % by mass;
the amount of the magnesium oxide: 50 to 90 % by mass; and
the amount of the inorganic oxide ZO: 5.0 to 40 % by mass.

The most preferable ranges of the amounts of the tungsten oxide, the magnesium oxide and the above-mentioned inorganic oxide ZO relative to 100% by mass of the synthesis catalyst are as follows:

the amount of the tungsten oxide: 3.0 to 10 % by mass;
the amount of the magnesium oxide: 70 to 85 % by mass; and
the amount of the inorganic oxide ZO: 10 to 20 % by mass.

When the amounts of the tungsten oxide, the magnesium oxide and the above-mentioned inorganic oxide ZO are within the above ranges, the synthesis efficiency of the target product can be further improved.

Examples of the above-mentioned inorganic oxide ZO include silicon oxides such as a composite dispersion composed of silicon and silicon dioxide represented by the general formula SiOx (wherein x represents a number of 0.5 or more and less than 1.6) (e.g., silicon monoxide (SiO)) and silicon dioxide ($SiO_2$). Further examples of the above-mentioned inorganic oxide ZO may include inorganic oxides such as diatomaceous earth, mica, zeolite, and silicoaluminophosphoric acid. Specifically, in order to improve the synthesis efficiency of the target product, it is preferable that the above-mentioned inorganic oxide ZO is silicon dioxide. Specific examples of silicon dioxide include colloidal silica (silica sol), fumed silica, and mesoporous silica.

The preferable ranges of the amounts of the tungsten oxide, the magnesium oxide and the silicon dioxide relative to 100% by mass of the synthesis catalyst are as follows:

the amount of the tungsten oxide: 0.1 to 89.9 % by mass;
the amount of the magnesium oxide: 10 to 90 % by mass; and
the amount of the silicon dioxide: 0.1 to 89.9 % by mass.

The more preferable ranges of the amounts of the tungsten oxide, the magnesium oxide and the silicon dioxide relative to 100% by mass of the synthesis catalyst are as follows:

the amount of the tungsten oxide: 1.0 to 45 % by mass;
the amount of the magnesium oxide: 30 to 90 % by mass; and
the amount of the silicon dioxide: 1.0 to 65 % by mass.

The still more preferable ranges of the amounts of the tungsten oxide, the magnesium oxide and the silicon dioxide relative to 100% by mass of the synthesis catalyst are as follows:

the amount of the tungsten oxide: 2.0 to 20 % by mass;
the amount of the magnesium oxide: 50 to 90 % by mass; and
the amount of the silicon dioxide: 5.0 to 40 % by mass.

The most preferable ranges of the amounts of the tungsten oxide, the magnesium oxide and the silicon dioxide relative to 100% by mass of the synthesis catalyst are as follows:

the amount of the tungsten oxide: 3.0 to 10 % by mass;
the amount of the magnesium oxide: 70 to 85 % by mass; and
the amount of the silicon dioxide: 10 to 20 % by mass.

When the amounts of the tungsten oxide, the magnesium oxide and the silicon dioxide are within the above ranges, the synthesis efficiency of the target product can be further improved.

Here, the amount of the tungsten oxide contained in the synthesis catalyst is calculated in terms of $WO_3$, the amount of the magnesium oxide is calculated in terms of MgO, and the amount of the silicon dioxide is calculated in terms of $SiO_2$.

When the amount of the tungsten oxide contained in the synthesis catalyst is within the above-mentioned range, the selectivity of 1,3-butadiene can be further improved. The tungsten oxide contained in an amount within the above range can prevent the tungsten oxide from being unevenly distributed on the surface of the catalyst or blocking the active sites formed by the magnesium oxide, thereby suppressing the decrease of the activity of the catalyst.

The magnesium oxide contained in the synthesis catalyst in an amount within the above-mentioned range can prevent the active sites formed by the magnesium oxide from decreasing, to thereby suppress the significant lowering of the yield of 1,3-butadiene. Further, the magnesium oxide contained in an amount within the above range can prevent the basicity of the catalyst from increasing, thereby suppressing the increase of the selectivity of n-butanol.

The silicon dioxide contained in the synthesis catalyst in an amount within the above-mentioned range can prevent the surface area of the catalyst from decreasing, which caused by agglomeration of crystals of the tungsten oxide or the magnesium oxide. As a result, the lowering of ethanol conversion can be suppressed. Further, the amount of the silicon dioxide contained in an amount within the above range can prevent the acid points of the catalyst from increasing, thereby suppressing the increase in the ethylene generation by dehydration of ethanol.

With respect to the above-mentioned inorganic oxides forming the synthesis catalyst, it is preferable that the inorganic oxides are uniformly dispersed in the synthesis catalyst. The uniform dispersion of the inorganic oxides results in uniformity in the above-mentioned amounts and ratios of the oxides throughout the catalyst, thereby enabling the target product to be produced further more efficiently. The expression "uniform" here means, for example, that there is no uneven distribution of the above-mentioned inorganic oxides in the synthesis catalyst particles.

Here, in the synthesis catalyst of the present embodiment, the inorganic oxide used as a carrier to be described later is unevenly distributed in the synthesis catalyst, and hence is not included in the definition of the catalyst components constituting the synthesis catalyst. For example, when silica (silicon dioxide) which is much larger in volume than the synthesis catalyst is used as a carrier, and the synthesis catalyst is produced by the impregnation method, the silica aggregates with each other to form a carrier and, hence, cannot be said to be uniformly dispersed in the synthesis catalyst even if the other oxides are uniformly dispersed on the silica carrier. Therefore, the silica constituting the carrier used in the impregnation method does not correspond to the silicon dioxide uniformly dispersed in the synthesis catalyst as the above-mentioned inorganic oxide ZO.

As a method for examining the distribution of the inorganic oxides in the synthesis catalyst of this embodiment, for example, a method employing an electronic probe microanalyzer (EPMA) can be mentioned. In general, the composition of the cross section of the analytical sample can be analyzed by EPMA and the distribution of each element in the cross section can be examined. When the inorganic oxides are uniformly distributed in the synthesis catalyst, the uniform distribution of the corresponding elements is confirmed by the surface analysis via EPMA with respect to, for example, an arbitrary cross section of a particle of the synthetic catalyst.

As the carrier, any of the known carriers used for catalysts can be used and, for example, porous carriers are preferable.

There is no particular limitation with respect to the material of the porous carrier, and examples thereof include silica, zirconia, titania, magnesia, alumina, activated carbon and zeolite. Among these, silica is preferable because various silica products differing in specific surface area and pore size are commercially available. However, when the catalyst contains silicon dioxide as the aforementioned inorganic oxide affecting the catalytic activity, it is preferable to use a carrier other than silica so as to preserve the effect of adjusting the amount of the silicon dioxide.

The size of the porous carrier is not particularly limited; however, the particle diameter thereof is preferably 0.5 to 5000 $\mu$m. The particle diameter more than the above lower limit value can prevent the porous carrier from scattering easily, or becoming difficult to handle. The particle diameter not more than the above upper limit value can prevent the degree of improvement of the synthesis efficiency from being decreased by the reduced amount supported catalyst components due to the increased difficulty in entry of the catalyst components into the carrier.

The particle size of the porous carrier can be controlled by sifting.

In addition, the porous carrier is preferably one with a particle size distribution as narrow as possible.

The total of the volume of the pores of the porous carrier (total pore volume) is not particularly limited and, for example, is preferably 0.01 to 1.0 mL/g, more preferably 0.1 to 0.8 mL/g, and still more preferably 0.3 to 0.7 mL/g. The total pore volume more than the above lower limit value can prevent the degree of improvement of the synthesis efficiency from being decreased by excessively small specific surface area of the porous carrier, which reduces the dispersibility of the catalyst metals in the process of causing the catalyst metals to be supported on the carrier. The total pore volume not more than the above upper limit value can prevent the degree of improvement of the synthesis efficiency from being decreased by the reduced amount supported catalyst components due to the increased difficulty in entry of the catalyst components into the carrier.

The total pore volume is a value measured by the aqueous titration method. In the aqueous titration method, water molecules are adsorbed onto a surface of the porous carrier, and the pore distribution is measured based on the condensation of the molecules.

The average pore diameter of the porous carrier is not particularly limited, and, for example, is preferably 0.01 to 20 nm, more preferably 0.1 to 8 nm. The average pore diameter not less than the above lower limit value can prevent the synthesis efficiency from being decreased by the reduced amount supported catalyst components due to the increased difficulty in entry of the catalyst components into the carrier, or by the reduced dispersibility of the catalyst components in the process of causing the catalyst components to be supported on the carrier. The average pore diameter not more than the above upper limit value can prevent the synthesis efficiency from being decreased by excessively small specific surface area of the carrier, which reduces the dispersibility of the catalyst components in the process of causing the catalyst components to be supported on the carrier.

The average pore diameter is a value measured by the following method. When the average pore diameter is at least as large as 0.1 nm, but is less than 10 nm, the average pore diameter is calculated from the total pore volume and the BET specific surface area. When the average pore diameter is at least as large as 10 nm, the average pore diameter is measured by the mercury penetration method using a mercury porosimeter.

Here, the total pore volume is a value measured by the aqueous titration method, and the BET specific surface area is a value calculated from an adsorbed amount of nitrogen that is an adsorption gas, and a pressure at the time of adsorption.

In the mercury penetration method, a pressure is applied to inject mercury into pores of the porous carrier, and the average pore diameter is calculated from the pressure and an amount of the mercury injected.

The specific surface area of the porous carrier is not particularly limited and, for example, is preferably 1 to 1000 $m^2$/g, more preferably 300 to 800 $m^2$/g, still more preferably 400 to 700 $m^2$/g. The specific surface area not less than the above lower limit value can prevent the synthesis efficiency from being decreased by excessively small specific surface area of the carrier, which reduces the dispersibility of the catalyst components in the process of causing the catalyst components to be supported on the carrier. The specific surface area not more than the above upper limit value can prevent the synthesis efficiency from being decreased by the reduced amount supported catalyst components due to the increased difficulty in entry of the catalyst components into the carrier, or by the reduced dispersibility of the catalyst components in the process of causing the catalyst components to be supported on the carrier.

The specific surface area means the BET specific surface area, which is measured by the BET gas adsorption method using nitrogen as an adsorption gas.

The amount of the supported catalyst component relative to 100 parts by mass of the carrier is preferably 0.5 parts by mass or more, more preferably 0.75 parts by mass or more, still more preferably 1.0 part by mass or more. When the amount is 0.5 parts by mass or more, the synthesis efficiency of the target product can be further improved.

The upper limit value of the amount of the supported catalyst components relative to 100 parts by mass of the carrier is determined in consideration of the specific surface area of the carrier or the like, and is preferably, for example, 100 parts by mass or less, more preferably 50 parts by mass or less. No further improvement in synthesis efficiency may be available even when the catalyst components are supported in an amount exceeding the above upper limit value.

Regardless of the presence or absence of the carrier, the synthesis catalyst preferably has a composition represented by the following formula (I).

$$a\,A \cdot b\,B \cdot c\,C \quad \cdots\cdots(I)$$

wherein A represents tungsten oxide, B represents magnesium oxide, C represents the above inorganic oxide ZO, a, b and c represent molar ratios, and a + b + c = 1.

In the formula (I), when c = 0, a may be 0.0001 to 0.05. When c > 0, a is preferably 0.0001 to 0.05, more preferably 0.0005 to 0.030, still more preferably 0.0010 to 0.015. The value of a not less than the above lower limit value can prevent the amount of the tungsten oxide from becoming too small to sufficiently increase the ethanol conversion. The value of a not more than the above upper limit value can prevent the amounts of other oxides from becoming too small to sufficiently increase the ethanol conversion.

In the formula (I), when c = 0, b may be 0.95 to 0.9999. When c > 0, b is preferably 0.55 to 0.98, more preferably 0.70 to 0.97, still more preferably 0.80 to 0.95. The value of b not less than the above lower limit value can prevent the amount of the magnesium oxide from becoming too small to sufficiently increase the ethanol conversion. The value of b not more than the above upper limit value can prevent the amounts of other oxides from becoming too small to sufficiently increase the ethanol conversion.

In the formula (I), c may either be 0 (that is, the above-mentioned inorganic oxide ZO is not present) or more than 0 (that is, the above-mentioned inorganic oxide ZO is present). When the synthesis catalyst contains the above-mentioned inorganic oxide ZO, c is preferably 0.01 to 0.40, more preferably 0.03 to 0.30, still more preferably 0.05 to 0.20. The value of c not less than the above lower limit value can prevent the amount of the inorganic oxide ZO from becoming too small to sufficiently increase the ethanol conversion. The value of c not more than the above upper limit value can prevent the amounts of other oxides from becoming too small to sufficiently increase the ethanol conversion.

The above molar fractions can be calculated on the assumption that, for example, the tungsten oxide is $WO_3$, the magnesium oxide is MgO, and the inorganic oxide ZO is $SiO_2$ in the synthesis catalyst.

As the synthesis catalyst of the present embodiment, for example, a composite oxide catalyst represented by the following composition formula (2) can be mentioned.

Composition formula (2): $W_\alpha Mg_\beta Z_\gamma O_\theta$

wherein Z represents one or more elements other than oxygen, which form the inorganic oxide ZO, $0<\alpha<1$, $0<\beta<1$, $0\leqq\gamma<1$, $0<\theta<1$, $A+\beta+\gamma+\theta\leqq1$.

In the formula (2), it is preferable that $\alpha < \beta$, and it is more preferable that

$$0.001 \leqq \alpha \leqq 0.01,$$

$$0.3 \leqq \beta <0.5,$$

$$0.001 \leqq \gamma \leqq 0.01,$$

and/or

$$0.5 \leqq \theta <1.$$

Here, $\alpha$, $\beta$, $\gamma$ and $\theta$ are atomic ratios (molar ratios).

When the synthesis catalyst does not have a carrier, it is preferable that the synthesis catalyst per se is porous. Such a porous unsupported-type composite oxide catalyst can be obtained by, for example, a kneading method to be described later.

<Method for Producing Synthetic Catalyst>

The synthesis catalyst of the present invention is produced by a known method such as a kneading method, an impregnation method, a vapor deposition method, a supported complex decomposition method or the like. An example of the method for producing the synthesis catalyst will be described below.

[Kneading Method]

In a method as one example of the embodiment, the oxides for forming the synthesis catalyst or precursors of the oxides are mixed in a dispersion medium or a solvent, followed by kneading with an automatic mill or the like, the obtained dispersion or solution is heated and dried, and the resultant product is calcinated. This method can provide a synthesis catalyst in which the oxides are uniformly dispersed.

Hereinafter, unless otherwise specified, the dispersion medium and the solvent are not distinguished from each other, and the dispersion and the solution are not distinguished from each other.

By adjusting the compounding ratio of the oxides and/or the precursors of the oxides when mixing in the dispersion medium, it is possible to obtain a synthesis catalyst with the amounts and ratios of the oxides as described above. For example, by setting the compounding ratio so as to correspond to the amounts and ratios of the oxides as described above, it is possible to obtain a synthesis catalyst with the amounts and ratios of the oxides according to the compounding ratio.

The precursors of the oxides are preferably compounds containing an oxygen atom (oxygen-containing precursors) because the oxides forming the synthesis catalyst can be easily obtained.

Examples of suitable precursor of the tungsten oxide include alkoxides of tungsten. As the alkoxide, methoxide or ethoxide is preferable.

Examples of suitable precursor of the magnesium oxide include hydroxides, nitrates, oxalates of magnesium. Among these, hydroxides are preferable since the oxides can be easily obtained by heating.

Examples of suitable precursor of other inorganic oxides other than tungsten oxide and magnesium oxide include colloidal silica. When colloidal silica is used, a porous composite oxide type catalyst can be easily obtained.

By adjusting the average particle diameter of the colloidal silica in this embodiment, a porous synthesis catalyst having a desired specific surface area can be obtained without using a carrier. The average primary particle diameter of the colloidal silica is not particularly limited. For example, the primary particle diameter is preferably about 1 nm to 200 nm. Colloidal silica preferably has high sphericity and is monodisperse. Such colloidal silica is commercially available.

By stirring the colloidal silica in water as a dispersion medium, the surface of the colloidal silica is covered with hydroxyl groups and interacts or reacts with other precursors via the hydroxyl groups. For example, when the colloidal silica is mixed with magnesium hydroxide in water, the magnesium hydroxide is uniformly distributed on the surface of colloidal silica due to affinity between respective hydroxyl groups possessed by both of the colloidal silica and the magnesium hydroxide. Further, for example, when mixed with an alkoxide of tungsten, the alkoxy groups thereof are hydrolyzed in water into hydroxyl groups, which is considered to allow the alkoxide to be uniformly distributed on the surface of colloidal silica as in the case of the hydroxides mentioned above. Therefore, when a ternary system of the colloidal silica, the hydroxide and the alkoxide are mixed in water, a sol in which the ternary system is uniformly mixed is obtained. After this mixing operation, a solid in which the ternary system is uniformly mixed is obtained by a drying operation with moderate heating and drying. By the subsequent calcination operation where the resulting dry solid is calcinated, a porous synthesis catalyst having excellent catalytic activity can be obtained.

The mixing method in the mixing operation is not particularly limited as long as it is a method capable of uniformly mixing the materials. For example, the mixing method may be a known method of stirring a dispersion medium containing the materials with a known mixer such as a mill, a mixer or a blender. Examples of the dispersion medium include water, methanol, ethanol, tetrahydrofuran, dioxane, hexane, benzene, toluene and mixtures thereof.

The temperature of the dispersion in the mixing operation is not particularly limited. For example, the temperature may be a moderate temperature of about 4 to 40 °C such that unnecessary chemical reactions such as an unintended condensation reaction, polymerization reaction and the like do not occur. The mixing time is not particularly limited as well. For example, the mixing time may be about 0.5 hour to 12 hours.

The drying method in the drying operation is not particularly limited. For example, the drying method may be a method of heating a sufficiently mixed dispersion at an arbitrary temperature. The heating temperature in the drying operation may be any temperature as long as the dispersion medium of the dispersion can be evaporated. For example, when the dispersion medium is water, the heating temperature is preferably 80 to 120 °C. For preventing unnecessary chemical reactions from occurring, a mild heating may be performed by a two-step drying method in which most of the dispersion medium is evaporated at a relatively low temperature of, for example, about 80 °C, and the remaining dispersion medium is evaporated at a relatively high temperature of about 110 °C.

The heating temperature in the calcination operation is, for example, preferably 300 to 600 °C. By performing the calcination operation, the components that do not contribute to the catalytic reaction among the components contained in the raw material compounds are sufficiently volatilized, the precursors of the respective oxides are changed into the respective oxides, and the oxides are sintered together, whereby the catalytic activity of the synthesis catalyst can be further enhanced.

Although the case of a ternary system has been described above, the same mixing operation, drying operation and calcination operation as described above can be employed for obtaining the synthesis catalyst even in the case of a binary system consisting of tungsten oxide and magnesium oxide without using the above-mentioned inorganic oxide such as colloidal silica.

[Impregnation Method]

In a method as one example of another embodiment, a porous carrier is impregnated with an impregnating solution containing the oxides forming the synthesis catalyst or the precursors of the oxides (impregnation operation), the carrier impregnated with the impregnating solution is dried (drying operation), and the dried carrier is calcinated at an arbitrary temperature (calcination operation).

The impregnation method in the impregnation operation is determined in consideration of the type of the carrier, and examples thereof include conventionally known methods such as a method of dropping or spraying an impregnating solution onto a carrier, followed by stirring, and a method of immersing a carrier in an impregnating solution.

The impregnating solution is one in which the oxides forming the synthesis catalyst or the precursors of the oxides are dispersed in a dispersion medium. Examples of the dispersion medium include water, methanol, ethanol, tetrahydrofuran, dioxane, hexane, benzene, toluene and mixtures thereof.

There is no particular limitation on the drying method in the drying operation, and examples thereof include a method in which the carrier impregnated with the impregnating solution is heated at an arbitrary temperature. The heating temperature in the drying operation may be any temperature as long as the dispersion medium of the impregnation solution can be evaporated. For example, when the dispersion medium is water, the heating temperature is preferably 80 to 120 °C.

The heating temperature in the calcination operation is, for example, preferably 300 to 600 °C. By performing the calcination operation, the components that do not contribute to the catalytic reaction among the components contained in the raw material compounds are sufficiently volatilized, the precursors of the respective oxides are changed into the respective oxides, and the oxides are sintered together, whereby the catalytic activity of the synthesis catalyst can be further enhanced.

<Apparatus for Producing Oxygenate>

The production apparatus 10 shown in FIG. 1 has a reaction tube 1 which is filled with the synthesis catalyst and has formed therein a reaction bed 2; a supply tube 3 connected with the reaction tube 1; a withdrawal tube 4 connected with the reaction tube 1; a temperature control unit 5 connected with the reaction tube 1; and a pressure control unit 6 provided at the withdrawal tube 4.

The reaction bed 2 may be either one charged only with the synthesis catalyst or one charged with a mixture of the synthesis catalyst and a diluent. Since the reaction of synthesizing 1,3-butadiene from ethanol is an endothermic reaction, a diluent for the purpose of preventing the synthesis catalyst from excessively generating heat is not normally necessary.

Examples of the diluent include those similar to the carrier for the synthesis catalyst, quartz sand, alumina ball, aluminum ball, and aluminum shot.

When the diluent is filled into the reaction bed 2, the mass ratio in terms of diluent/synthesis catalyst may be determined in view of the types and the specific gravity and the like of the diluent and the catalyst and is, for example, preferably 0.5 to 5.

The reaction tube 1 is preferred to be made of a material which is inert to the gaseous raw material and the synthesized product and is preferred to have a shape such that the reaction tube 1 can withstand a heating at around 100 to 500 °C and a pressure of around 10 MPa.

As a specific example of the reaction tube 1, there can be mentioned an approximately cylindrical part made of a stainless steel.

The supply tube 3 is a means for supplying a gaseous raw material to the reaction tube 1, and may be, for example, a pipe made of a stainless steel etc.

The withdrawal tube 4 is a means for withdrawing a gas containing a product synthesized in the reaction bed 2, and may be, for example, a pipe made of a stainless steel etc.

With respect to the temperature control unit 5, there is no particular limitation as long as it can control the temperature of the reaction bed 2 in the reaction tube 1 to a desired value, and examples of the temperature control unit 5 include an electric furnace and the like.

With respect to the pressure control unit 6, there is no particular limitation as long as it can control the internal pressure of the reaction tube 1 to a desired value, and examples of the pressure control unit 6 include a known pressure valve or the like.

The production apparatus 10 may be equipped with a known device such as a gas flow rate controller (e.g., mass flow controller) or the like which adjusts a flow rate of the gas.

<Method for Producing 1,3-Butadiene>

1,3-Butadiene can be produced by contacting ethanol with the above-mentioned synthesis catalyst. In the production method of the present embodiment, it is preferable that the ethanol to be contacted with the synthesis catalyst is in a vaporized state, and it is more preferable that the vaporized ethanol contacts with the synthesis catalyst while being heated. Hereinafter, an example of the method for producing 1,3-butadiene using the production apparatus shown in FIG. 1 will be described.

First, the temperature and pressure in the reaction tube 1 are adjusted to predetermined values, and the gaseous mixture 20 containing vaporized ethanol is introduced into the reaction tube 1 through the supply tube 3.

The gaseous raw material 20 may be composed only of an ethanol gas or a mixed gas 20 containing an ethanol gas and a gas other than ethanol gas.

The mixed gas 20 may contain an inert gas. When the mixed gas 20 contains an inert gas, the synthesis efficiency of 1,3-butadiene can be further improved.

With respect to the lower limit value of the inert gas content of the mixed gas 20, for example, the content is preferably 5 % by volume or more, more preferably 10 % by volume or more, still more preferably 15 % by volume or more. With the inert gas content more than the lower limit value, the effect of incorporating the inert gas into the mixed gas 20 is further enhanced, thereby further improving the synthesis efficiency of 1,3-butadiene.

With respect to the upper limit value of the inert gas content of the mixed gas 20, for example, the content is preferably 50 % by volume or less, more preferably 40 % by volume or less, still more preferably 30 % by volume or less. With the inert gas content not more than the upper limit value, the amount of ethanol in the mixed gas 20 is sufficient so as to further improve the synthesis efficiency of 1,3-butadiene.

The ethanol in the gaseous raw material 20 may be "bioethanol" derived from biomass or ethanol derived from fossil fuel.

In addition to ethanol and inert gas, the mixed gas 20 may contain hydrogen, carbon monoxide, methane, ethane, ethylene, water and the like (hereinafter, these may be collectively referred to as gas impurities). However, from the viewpoint of further improving the synthesis efficiency of 1,3-butadiene, it is preferable not to allow the gas impurities to be contained in the mixed gas 20.

The temperature at which the gaseous raw material 20 and the catalyst are to be brought into contact (reaction temperature), namely the temperature inside the reaction tube 1, is, for example, preferably within a range from 150 to 500 °C, more preferably from 250 to 500 °C, still more preferably from 350 to 450 °C. When the reaction temperature is not lower than the above lower limit value, the rate of catalytic reaction can be sufficiently increased to enable 1,3-butadiene to be produced more efficiently. When the reaction temperature is not higher than the upper limit value, the 1,3-butadiene synthesis reaction becomes predominant, thereby further improving the selectivity for 1,3-butadiene.

The pressure (reaction pressure) at the time of bringing the gaseous raw material 20 into contact with the catalyst, namely the pressure inside the reaction tube 1, is preferably atmospheric pressure (normal pressure), but may be an elevated pressure. Specifically, for example, the pressure is preferably 0.1 MPa to 10 MPa, more preferably 0.5 MPa to 5.0 MPa, still more preferably 1.0 MPa to 2.5 MPa. When the reaction pressure is not lower the above-mentioned lower limit value, the rate of catalytic reaction can be sufficiently increased so as to further improve the synthesis efficiency of 1,3-butadiene. When the reaction pressure is not higher than the upper limit value, the 1,3-butadiene synthesis reaction becomes predominant, thereby further improving the synthesis efficiency of 1,3-butadiene.

The inflowing gaseous raw material 20 circulates while coming into contact with the catalyst of the reaction bed 2, and a part thereof becomes 1,3-butadiene. It is considered that the ethanol in the gaseous raw material 20 becomes 1,3-butadiene by a catalytic reaction represented by the following formula ($\alpha$).

$$2C_2H_5OH \rightarrow H_2C=CH-CH=CH_2 + 2H_2O + N_2 \cdots \qquad (\alpha)$$

Then, the resultant product gas 22 containing 1,3-butadiene is withdrawn from the withdrawal tube 4. The product gas 22 may contain C2 to C4 compounds such as acetaldehyde, ethylene, butene, propylene, and ethane.

The supply rate of the gaseous raw material 20 is, for example, preferably 10 to 100,000 L/L-catalyst/h in terms of the space velocity of the gaseous raw material (calculated as a standard state value thereof) in the reaction bed 2 (the value obtained by dividing the gas supply volume per unit time by the amount of the catalyst (in terms of volume)). The space velocity is adjusted as appropriate, with due consideration of the reaction pressure, the reaction temperature and the composition of the gaseous raw material.

The contact time between the gaseous raw material 20 and the reaction bed 2 (synthesis catalyst) is, for example, preferably 1 to 50 seconds, more preferably about 3 to 20 seconds, still more preferably about 5 to 10 seconds.

When the contact time is too short, ethanol cannot be converted to butadiene while the yield of other products tends to increase.

On the other hand, when the contact time is too long, condensation and polymerization of acetaldehyde and butadiene proceed, which may result in an increased generation of high-boiling point components. The contact time can be controlled by adjusting the above space velocity.

If necessary, the product gas 22 withdrawn from the withdrawal tube 4 may be processed in a gas-liquid separator or the like to separate the reaction product from the unreacted gaseous raw material 20. Further, the reaction product may be purified by a separation means such as filtration, concentration, distillation, and extraction.

In the present embodiment, the gaseous raw material is brought into contact with the reaction bed 2 that is a fixed bed, but the synthesis catalyst may also be provided in other form than a fixed bed, for example, in the form of a fluidized bed, a moving bed or a suspended bed, with which the gaseous raw material is contacted.

The synthesis catalyst of the present invention has a structure in which the oxides which are active species of the catalyst are bonded to each other, which can suppress the elution of the active species into the reaction solution or the diffusion of the active species into the reaction gas even during the catalytic reaction. Therefore, the method for producing 1,3-butadiene according to the present embodiment may be either a gas phase method or a liquid phase method. From the viewpoint of ease of recovery of the catalyst and regeneration treatment of the recovered catalyst, it is preferably to employ a gas phase method in which the synthesis catalyst is filled in a reaction tube to form a catalyst layer, and the gaseous raw material is caused to flow therein to advance the reaction. When the liquid phase method is employed, the recovery of the synthesis catalyst can be carried out by a physical separation method such as filtration from a reaction solution, and centrifugation. Further, ethanol as an unreacted raw material may be recovered and reused.

Further, the method for producing 1,3-butadiene according to the present embodiment may be performed following any conventional process such as a batchwise process, a semi-batchwise process or a continuous process. The batchwise process or the semi-batchwise process can increase the utilization ratio of raw material ethanol. Further, even when a continuous system is employed, a higher ethanol conversion than conventional techniques is achievable due to the excellent catalytic activity of the above-mentioned synthesis catalyst, and recycling unreacted ethanol back into the reaction system can increase the utilization ratio of the raw material ethanol. From the viewpoint of easy and efficient separation and recovery of 1,3-butadiene, it is preferred to employ the continuous process.

The synthesis catalyst used in the method of the present embodiment can be allowed to regain its catalytic activity by flowing air in a reaction vessel while heating, for example, at about 150 to 500 °C for 0.1 to 24 hours, and used again for the 1, 3-butadiene synthesis reaction.

By the method for producing 1,3-butadiene according to the present embodiment, 1,3-butadiene can be produced selectively. For example, when a reaction is carried out at a reaction temperature of 420 °C, a reaction pressure of 0.1 MPa and a space velocity of 500 L / L-catalyst / h, the selectivity for 1,3-butadiene at 120 minutes after the start of the reaction is, for example, 40 % or more, preferably 50 % or more, more preferably 60 % or more. Further, the method for producing 1,3-butadiene according to the present embodiment can achieve excellent ethanol conversion. For example, when a reaction is carried out at a reaction temperature of 420 °C, a reaction pressure of 0.1 MPa and a space velocity of 500 L / L-catalyst / h, the ethanol conversion at 120 minutes after the start of the reaction is, for example, 74 % or more, preferably 85 % or more, more preferably 95 % or more.

As described above, 1,3-butadiene can be synthesized more efficiently by using the synthesis catalyst of the present invention.

EXAMPLES

Hereinbelow, the present invention will be described with reference to Examples which, however, should not be construed as limiting the present invention.

<Preparation of Catalyst>

(Example 1)

20.65 g of magnesium hydroxide Mg (OH)$_2$ (manufactured by Kanto Chemical Co., Inc.), 12.64 g of colloidal silica ("Snowtex XS (trade name)" manufactured by Nissan Chemical Industries; average primary particle diameter: 4 to 6 nm; surface area: 800 ± 200 m$^2$/g) and 1.56 g of tungsten ethoxide were suspended in water, followed by kneading for 3 hours by an automatic mill to obtain a sol. The obtained sol was dried by heating at 80 °C for 16 hours and then at 110 °C for 4 hours, and subsequently the reaction temperature was increased to 500 °C at a rate of 1 °C /min, followed by calcination at 500 °C for 2 hours to obtain a cake. The obtained cake was crushed and classified to obtain a catalyst (1) formed of porous particles having a particle diameter of 1.0 to 2.0 mm (MgO /MgO /WO3/ SiO$_2$ = 80.7 / 5.0 / 14.3 (mass ratio)).

(Comparative Example 1)

24.1 g of magnesium hydroxide Mg (OH)$_2$ (manufactured by Kanto Chemical Co., Inc.), 14.4 g of colloidal silica ("Snowtex XS (trade name)" manufactured by Nissan Chemical Industries; average primary particle diameter: 4 to 6 nm; surface area: 800 ± 200 m$^2$ / g) and 0.74 g of tungsten ethoxide were suspended in water, followed by kneading for 3 hours by an automatic mill to obtain a sol. The obtained sol was dried by heating at 80 °C for 16 hours and then at 110 °C for 4 hours, and subsequently the reaction temperature was increased to 500 °C at a rate of 1 °C /min, followed by calcination at 500 °C for 2 hours to obtain a cake. The obtained cake was crushed and classified to obtain a catalyst (2) formed of porous particles having a particle size of 1.0 to 2.0 mm (MgO / Ta$_2$O$_5$ / SiO$_2$ = 83.0 / 2.0 / 15.3 (mass ratio)).

<Method for Synthesizing 1,3-Butadiene>

(Example 1)

3.82 g of the obtained catalyst (1) was filled into a cylindrical reaction tube made of stainless steel having a diameter of 1.5 inches (1.27 cm) and a length of 10 inches (25.4 cm) to form a reaction bed.

Subsequently, with the temperature of the reaction bed and the reaction temperature being kept at 420 °C, a mixed gas described below was allowed to flow through the reaction bed at a space velocity of 500 L / L-catalyst / h and atmospheric pressure to obtain a product gas containing 1,3-butadiene.

The mixed gas was allowed to flow through the reaction tube under the condition that the mixed gas (ethanol: 70 % by volume, nitrogen: 30 % by volume) contacts the reaction bed for 7.2 seconds, whereafter the obtained product gas was recovered and analyzed by gas chromatography.

From the obtained data, the ethanol conversion (mol%), the 1,3-butadiene selectivity (mol%), the 1,3-butadiene yield (mol%), the acetaldehyde selectivity (mol%), the ethylene selectivity (mol%), the butene selectivity (mol%), the propylene selectivity (mol%), and the ethane selectivity (mol%) were calculated. The results are shown in Table 1.

(Comparative Example 1)

3.87 g of the obtained catalyst (2) was filled into a cylindrical reaction tube made of stainless steel having a diameter of 1.5 inches (1.27 cm) and a length of 10 inches (25.4 cm) to form a reaction bed.

Subsequently, with the temperature of the reaction bed and the reaction temperature being kept at 420 °C, a mixed gas described below was allowed to flow through the reaction bed at a space velocity of 500 L / L-catalyst / h and atmospheric pressure to obtain a product gas containing 1,3-butadiene.

The mixed gas was allowed to flow through the reaction tube under the condition that the mixed gas (ethanol: 70 % by volume, nitrogen: 30 % by volume) contacts the reaction bed for 7.2 seconds, whereafter the obtained product gas was recovered and analyzed by gas chromatography. The results of calculations carried out in the same manner as in Example 1 are shown in Table 1.

[Table 1]

| | Catalyst (mass ratio) | Mixed gas composition (molar ratio) | Contact time (Sec) | Ethanol conversion (%) | 1,3-Butadiene selectivity (%) | 1,3-Butadiene yield (%) | Acetaldehyde selectivity (%) | Ethylene selectivity (%) | Butene selectivity (%) | Propylene selectivity (%) | Ethane selectivity (%) |
|---|---|---|---|---|---|---|---|---|---|---|---|
| Example 1 | $MgO/WO_3/SiO_2$ (80.7/5.0/14.3) | ethanol/ nitrogen (70/30) | 7.2 | 95.5 | 60.6 | 57.8 | 1 | 24.5 | 7.7 | 5.4 | 0.8 |
| Comparative Example 1 | $MgO/Ta_2O_5/SiO_2$ (83.0/2.0/15.0) | ethanol/ nitrogen (70/30) | 7.2 | 71.8 | 56 | 40.2 | 5.2 | 31.3 | 4.1 | 2.9 | 0.5 |

In Table 1, the yield (%) of 1,3-butadiene in Example 1 is calculated as the product of 1,3-butadiene selectivity (%) × 0.955 (ethanol conversion). The calculation is likewise carried out for Comparative Example 1. The total of the selectivities for the respective products (oxygenates) is 100 %. It is clear from Table 1 that the ethanol conversion in Example 1 is especially remarkably high. Further, the selectivity for and the yield of 1,3-butadiene in Example 1 are also excellent.

The elements, combinations thereof, etc. that are explained above in connection with the specific embodiments of the present invention are mere examples, and various alterations such as addition of conventional element, omission and substitution of any elements, etc. may be made as long as such alterations do not deviate from the gist of the present invention. Further, the present invention is in no way limited by these embodiments.

DESCRIPTION OF THE REFERENCE SIGNS

1    Reaction tube
2    Reaction bed
3    Supply tube
4    Withdrawal tube
5    Temperature control unit
6    Pressure control unit
10   Production apparatus
20   Gaseous raw material
22   Product gas

Claims

1. A catalyst for synthesizing 1,3-butadiene by contact with ethanol, which comprises tungsten oxide and magnesium oxide.

2. The catalyst according to claim 1, wherein a mass ratio of the magnesium oxide to the tungsten oxide (magnesium oxide / tungsten oxide) is 0.1 to 200.

3. The catalyst according to claim 1 or 2, wherein the mass ratio is at least 5.

4. The catalyst according to any one of claims 1 to 3, wherein amounts of the tungsten oxide and the magnesium oxide relative to 100% by mass of the catalyst are as follows:

   the amount of the tungsten oxide: 0.1 to 90 % by mass; and
   the amount of the magnesium oxide: 10 to 90 % by mass.

5. The catalyst according to any one of claims 1 to 3, which further comprises at least one inorganic oxide other than tungsten oxide and magnesium oxide.

6. The catalyst according to claim 5, wherein an amount of the inorganic oxide other than tungsten oxide and magnesium oxide is 0.1 to 89.9 % by mass relative to 100 % by mass of the catalyst.

7. The catalyst according to claim 6, wherein amounts of the tungsten oxide, the magnesium oxide, and the inorganic oxide other than tungsten oxide and magnesium oxide relative to 100% by mass of the catalyst are as follows:

   the amount of the tungsten oxide: 0.1 to 89.9 % by mass;
   the amount of the magnesium oxide: 10 to 90 % by mass; and
   the amount of the inorganic oxide other than tungsten oxide and magnesium oxide: 0.1 to 89.9 % by mass.

8. The catalyst according to claim 6 or 7, wherein the inorganic oxide other than tungsten oxide and magnesium oxide is silicon dioxide.

9. A method for producing the catalyst according to any one of claims 1 to 8, comprising:

a step of mixing said oxides for constituting the catalyst or precursors of said oxides in a dispersion medium or a solvent to thereby obtain a dispersion or a solution;
a step of heating and drying the obtained dispersion or solution; and
a step of calcining the resultant dispersion or solution.

10. An apparatus for producing 1,3-butadiene, comprising:

a reaction tube filled with the catalyst according to any one of claims 1 to 8;
a supply means for supplying ethanol into the reaction tube; and
a withdrawal means for withdrawing a reaction product from the reaction tube.

11. A method for producing 1,3-butadiene, comprising contacting ethanol with the catalyst according to any one of claims 1 to 8.

FIG. 1

## INTERNATIONAL SEARCH REPORT

| International application No. |
|---|
| PCT/JP2016/053948 |

**A. CLASSIFICATION OF SUBJECT MATTER**
*B01J23/30*(2006.01)i, *B01J37/04*(2006.01)i, *C07C1/20*(2006.01)i, *C07C11/167*
(2006.01)i, *C07B61/00*(2006.01)n

According to International Patent Classification (IPC) or to both national classification and IPC

**B. FIELDS SEARCHED**

Minimum documentation searched (classification system followed by classification symbols)
B01J23/30, B01J37/04, C07C1/20, C07C11/167, C07B61/00

Documentation searched other than minimum documentation to the extent that such documents are included in the fields searched
Jitsuyo Shinan Koho      1922–1996   Jitsuyo Shinan Toroku Koho   1996–2016
Kokai Jitsuyo Shinan Koho   1971–2016   Toroku Jitsuyo Shinan Koho   1994–2016

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2013/125389 A1 (Daicel Corp.),<br>29 August 2013 (29.08.2013),<br>paragraphs [0001], [0006], [0007] to [0016],<br>[0023] to [0024], [0028], [0037], [0049],<br>[0110]<br>(Family: none) | 1–11<br>9 |
| X<br>Y | EP 2712673 A1 (LANXESS DEUTSCHLAND GMBH),<br>02 April 2014 (02.04.2014),<br>paragraphs [0001], [0011], [0013] to [0014],<br>[0017] to [0019], [0021] to [0022], [0024],<br>[0030] to [0056], [0059], [0078], [0080],<br>[0082] to [0084]; fig. 1<br>& WO 2014/049158 A1 | 1–8,10,11<br>9 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 March 2016 (22.03.16) | 05 April 2016 (05.04.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
|---|---|

Form PCT/ISA/210 (second sheet) (January 2015)

**INTERNATIONAL SEARCH REPORT**

International application No.

PCT/JP2016/053948

C (Continuation).   DOCUMENTS CONSIDERED TO BE RELEVANT

| Category* | Citation of document, with indication, where appropriate, of the relevant passages | Relevant to claim No. |
|---|---|---|
| A | JP 49-014405 A  (Ube Industries, Ltd.),<br>07 February 1974 (07.02.1974),<br>claims; page 3, upper left column, line 18 to<br>lower right column, line 4<br>& US 3895051 A            & GB 1414733 A<br>& DE 2320231 A1           & FR 2181975 A<br>& IT 982815 B | 1-11 |
| A | US 2423681 A  (ROHM & HAAS CO.),<br>08 July 1947 (08.07.1947),<br>the entire specification<br>(Family: none) | 1-11 |

Form PCT/ISA/210 (continuation of second sheet) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015038403 A
- JP 2013053211 A
- JP 2014129248 A